(19) Europäisches Patentamt / European Patent Office / Office européen des brevets

(11) **EP 1 541 122 B1**

(12) **FASCICULE DE BREVET EUROPEEN**

(45) Date de publication et mention
de la délivrance du brevet:
**19.01.2011 Bulletin 2011/03**

(51) Int Cl.:
*A61Q 15/00* (2006.01)     *A61K 8/26* (2006.01)
*A61K 8/49* (2006.01)     *A61K 8/27* (2006.01)

(21) Numéro de dépôt: **04292721.0**

(22) Date de dépôt: **17.11.2004**

(54) **Composition cosmétique déodorante comprenant l'association du pidolate de zinc et d'un sel d'aluminium anti-transpirant**

Desodorierende kosmetische Zusammensetzung enthaltend eine Assoziation von Zinkpidolat mit einem schweisshemmenden Aluminiumsalz

Cosmetic deodorant composition comprising an association of zinc pidolate with an anti-perspirant aluminium salt

(84) Etats contractants désignés:
**AT BE BG CH CY CZ DE DK EE ES FI FR GB GR HU IE IS IT LI LU MC NL PL PT RO SE SI SK TR**

(30) Priorité: **12.12.2003 FR 0351035**

(43) Date de publication de la demande:
**15.06.2005 Bulletin 2005/24**

(73) Titulaire: **L'Oréal**
**75008 Paris (FR)**

(72) Inventeurs:
• **Lemoine, Cyril**
**95380 Puiseux-en-France (FR)**
• **Beau, Nathalie**
**95610 Eragny-sur-Oise (FR)**
• **Prud'homme, Estelle**
**75018 Paris (FR)**

(74) Mandataire: **Miszputen, Laurent**
**L'Oréal**
**D.I.P.I.**
**25-29 Quai Aulagnier**
**92600 Asnières (FR)**

(56) Documents cités:
EP-A- 0 768 080     WO-A-00/68369
DE-A- 10 137 901     FR-A- 2 815 856
US-A- 5 643 559     US-B1- 6 403 067

Remarques:
Le dossier contient des informations techniques présentées postérieurement au dépôt de la demande et ne figurant pas dans le présent fascicule.

**Description**

L'invention a pour objet une composition cosmétique déodorante comprenant au moins :

a) le pidolate de zinc et
b) au moins un sel d'aluminium anti-transpirant particulier ; le rapport en poids pidolate de zinc/sel d'aluminium allant de 1/20 à 5/1.

L'invention concerne également un procédé de traitement cosmétique de la transpiration humaine et des odeurs axillaires humaines à l'aide de cette composition.

Dans le domaine cosmétique, il est bien connu d'utiliser en application topique, des produits déodorants contenant des substances actives de type anti-transpirant de type bactéricide ou du type absorbeur d'odeurs pour diminuer, voire supprimer, les odeurs axillaires généralement désagréables.

Les substances bactéricides inhibent le développement de la flore cutanée responsable des odeurs axillaires. Ils ont pour inconvénient de n'être pas actifs sur l'odeur de la sueur déjà développée. Parmi les produits bactéricides, le plus couramment employé est le Triclosan (2,4,4'-trichloro-2'-hydroxydiphényléther), qui présente l'inconvénient de modifier de façon importante l'écologie de inhibé par certains composés, comme par exemple les tensio-actifs non-ioniques, couramment utilisés dans la formulation de compositions cosmétiques. Par ailleurs, le caractère insoluble du Triclosan dans l'eau ne permet pas son incorporation dans des formules essentiellement aqueuses.

Les substances anti-transpirantes ont pour effet de limiter le flux sudoral. Elles sont généralement constituées de sels d'aluminium. Leur efficacité déodorante est limitée lorsqu'ils sont utilisés seuls. De plus, dans des concentrations élevées ces susbstances présentent un potentiel irritant pour la peau.

On connait dans le brevet DE 101 37901 l'utilisation d'inhibiteurs de l'arylsulfatase pour réduire la décomposition des esters de stéroïdes causant des odeurs corporelles.

On connaît également dans le brevet EP 768 080 des compositions déodorantes aqueuses en particulier des émulsions eau/silicone contenant comme actif absorbeur d'odeurs des sels de zinc hydrosolubles comme le pyrrolidone carboxylate de zinc appelé aussi pidolate de zinc, le sulfate de zinc, le chlorure de zinc, le pidolate de zinc est le phénolsulfonate de zinc. Leur efficacité déodorante n'est pas encore pleinement satisfaisante lorsqu'ils sont utilisés seuls.

On connaît également dans le brevet US 6,426,061 des compositions pour combattre le développement des odeurs de la transpiration humaine comprenant l'association des actifs suivants (1) un inhibiteur de récepteur androgène (resvératrol, epigallocatéchine-3-gallate ou acide flufénamique) ; (2) un actif anti-DHT (sels de zinc en particulier le sulfate de zinc) (3) un inhibiteur de protéines porteuses d'odeurs, (4) un sel d'aluminium anti-transpirant et (5) un agent antimicrobien du type chlorhexidine dipidolate ou chlorhexidine diacétate. Ces compositions présentent l'inconvénient d'utiliser des agents antimicrobiens particulièrement actifs sur la flore cutanée.

On connaît dans la demande de brevet WO01/52804 des compositions déodorantes à base de sels antitranspirants auxquels on propose de rajouter des chélatants de métaux de transition. Ces formulations sont potentiellement écotoxiques et posent des problèmes d'environnement.

On connaît également dans la demande de brevet WO01/99376 des compositions déodorantes contenant des inhibiteurs d'arylsulfatase parmi lesquels sont mentionnés les sels d'aluminium et pidolate de zinc. Ce document décrit en particulier un exemple de stick anti-transpirant contenant 20% en poids chlorhydrate et 0,05% de pidolate de zinc. Ce type de composition produit un taux élevé de résidu blanc sur la peau après application

Le but de la présente invention est donc de disposer de nouvelles compositions cosmétiques comprenant système déodorant ayant une efficacité supérieure à celle des sels anti-transpirants et à celle des sels de zinc utilisés seuls et qui ne présentent pas les inconvénients des produits déodorants de l'art antérieur tel qu'évoqués précédemment.

La demanderesse a découvert qu'une telle composition est obtenue en utilisant l'association du pidolate de zinc (ou pyrrolidone carboxylate de zinc) et d'un sel d'aluminium antitranspirant particulier en poids pidolate de zinc /sel d'aluminum variant de 1/20 à 5/1.

La demanderesse a découvert de manière inattendue que cette association particulière augmentait de manière substantielle l'effet de diminution de l'intensité de l'odeur par rapport aux actifs utilisés individuellement et pouvait même produire un effet de synergie

Elle a également découvert que cette association particulière pouvait être formulée dans des compositions déodorantes cosmétiquement acceptables dont les teneurs en résidu visible sur la peau à l'application ou après séchage de la composition après l'application sont faibles et comparables aux produits déodorants actuellement sur le marché.

De façon plus précise, l'invention a pour objet une composition cosmétique déodorante, comprenant au moins.

a) le pidolate de zinc et
b) au moins le chlorhydrate d'aluminium sous forme activée ou non activée le rapport en poids pidolate de zinc / sel d'aluminium allant de 1/20 à 5/1

L'invention concerne également un procédé de traitement cosmétique de la transpiration humaine et des odeurs axillaires humaines à l'aide de cette composition.

Au sens de la présente invention, on entend par "composition déodorante" toute composition capable de réduire le flux sudoral, masquer, absorber, améliorer et/ou réduire l'odeur désagréable résultant de la décomposition de la sueur humaine par des bactéries.

Les sels d'aluminium anti-transpirants peuvent être présents dans la composition selon l'invention à raison d'environ 0,5 à 25% en poids par rapport au poids total de la composition.

Le pidolate de zinc peut être présent dans la composition selon l'invention à raison d'environ 0,05 % à 10% en poids et plus préférentiellement de 0,1 à 5% en poids par rapport au poids total de la composition.

Les compositions déodorantes selon l'invention destinées à l'usage cosmétique peuvent se présenter sous forme de lotions, de crèmes ou de gels fluides distribués en spray aérosol, en flacon pompe ou en roll-on, sous forme de crèmes épaisses distribuées en tubes ou en grille ; sous forme de bâtonnets (sticks), et contenir à cet égard les ingrédients généralement utilisés dans ce type de produits et bien connus de l'homme de l'art, sous réserve qu'ils n'interfèrent pas avec le sel d'aluminium et le pidolate de zinc décrit dans la présente invention.

Les compositions déodorantes selon l'invention destinées à l'usage cosmétique peuvent comporter au moins une phase aqueuse. Elle sont notamment formulées en lotions aqueuses ou en émulsion eau-dans-huile, huile-dans-eau, ou en émulsion multiple (émulsion triple huile-dans-eau-dans-huile ou eau-dans-huile-dans-eau (de telles émulsions sont connues et décrites par exemple par C. FOX dans « Cosmétics and Toiletries » - november 1986-Vol 101-pages 101-112).

La phase aqueuse desdites compositions contient de l'eau et en général d'autres solvants solubles ou miscibles dans l'eau. Les solvants solubles ou miscibles dans l'eau comprennent les mono alcools à chaîne courte par exemple en $C_1$-$C_4$ comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.

Selon une forme particulière de l'invention, les compositions anti-transpirantes peuvent être anhydres.

Par "anhydre", on entend au sens de l'invention, une composition dont la teneur en eau libre ou ajoutée est inférieure à 3% et de préférence dont la teneur en eau ajoutée est inférieure à 1% en poids par rapport au poids total de la composition.

Les compositions selon l'invention comprennent de préférence au moins une phase liquide organique non-miscible dans l'eau Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase organique liquide organique non-miscible dans l'eau conforme à l'invention est généralement constituée d'une huile ou de mélange d'huiles et comprend au moins 80% de composés ayant une vapeur de pression ne dépassant pas la valeur 4 kPa (30 mm Hg) à 25°C.

La phase liquide organique non-miscible dans l'eau contient de préférence une ou plusieurs huiles émollientes siliconée ou hydrocarbonée, volatiles ou non-volatiles. Ces huiles émollientes sont notamment décrites dans les brevets US 4,822,596 et US 4,904,463.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones $D_4$, $D_5$ ou $D_6$.

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 245 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.

Parmi les huiles émollientes non-volatiles utilisables dans la présente invention, on peut citer par exemple : les dérivés hydrocarbonés, les huiles minérales, les alcools gras, les esters d'alcools en $C_3$-$C_{18}$ avec des acides en $C_3$-$C_{18}$, les esters de l'acide benzoïque avec des alcools en $C_{12}$-$C_{18}$ et leurs mélanges, des polyols en $C_2$-$C_6$ choisis de préférence parmi le glycérol, le propylèneglycol ou le sorbitol, les polymères de polalkylène glycol.

Les huiles émollientes sont présentes de préférence dans des quantités allant de 1 à 50% en poids et plus préférentiellement de 5 à 40% en poids par rapport au poids total de la composition.

La composition cosmétique déodorante selon l'invention peut contenir un ou plusieurs actifs déodorants additionnels comme par exemple des agents bactériostatiques ou des agents bactéricides tels que le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol , monolaurate de glycérol ; les sels de polyhexaméthylène biguanide.

Afin d'améliorer l'homogénéité du produit, on peut utiliser en plus un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une poudre organique.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 $\mu$m et masse volumique 40 kg/m3), 551 DE 20 (granulométrie d'environ 30 $\mu$m et masse volumique 65 kg/m3), 551 DE 50 (granulométrie d'environ 40 $\mu$m), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyllysine commercialisée sous la dénomination AMIHOPE LL-11 par la Société Ajinomoto ; les particules de microdispersion de cire, qui ont de préférence des dimensions moyennes inférieures à 1 $\mu$m et notamment allant de 0,02 $\mu$m à 1 $\mu$m, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges. La ou les poudres organiques peuvent être présentes par exemple en une quantité

La composition cosmétique selon l'invention peut comprendre en outre des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les cires peuvent être choisies parmi les cires animales, fossiles, végétales, minérales ou de synthèse. On peut citer notamment les cires d'abeille, les cires de Carnauba, de Candellila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MIO vendue par la société NL INDUSTRIES ou la Veegum Ultra, vendue par la société POLYPLASTIC.

Les quantités de ces différents constituants pouvant être présents dans la composition cosmétique selon l'invention sont celles classiquement utilisées dans compositions déodorantes.

Les compositions selon l'invention peuvent également contenir également un ou plusieurs autres agents structurants ou gélifiants de la phase liquide organique non-miscible dans l'eau de la composition tels que les alcools gras solides linéaires et/ou les cires ; les acides gras ou leurs sels (acide stérique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'-dibutylsuccinamide) ;les organopolysiloxane élastomères tels que ceux décrits dans la demande WO97/44010.

La composition selon l'invention peut encore être pressurisée et être conditionnée dans un dispositif aérosol.

La présente invention a pour objet un dispositif aérosol constitué par :

(A) un récipient comprenant une composition déodorante telle que définie précédemment,
(B) au moins un agent propulseur et un moyen de distribution de la dite composition aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

La composition contenant le ou les actifs déodorants et le ou les agents propulseurs peuvent se trouver dans le même compartiment ou dans des compartiments différents dans le récipient aérosol. Selon l'invention, la concentration en agent propulseur varie généralement de 5 à 95% en poids pressurisée et plus préférentiellement de 50 à 85% en poids par rapport au poids total de la composition pressurisée.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

La présente invention a également pour objet un procédé cosmétique pour traiter les odeurs axillaires humaines, consistant à appliquer sur la surface axillaire une quantité efficace d'une composition telle que définie ci-dessus.

Les exemples qui suivent servent à illustrer la présente invention.

## I/ Comparaison de l'activité déodorante de l'association pidolate de zinc/sel d'aluminium par rapport aux association triclosan/sel d'aluminium et ricinoleate de zinc /sel d'aluminium.

Protocole du test d'efficacité déodorante

Des recueils de sueur axillaire sont effectués en sauna sur 6 volontaires, les échantillons de sueur individuelle, conservés dans la glace pendant quelques heures, sont alors pratiquement inodores. Ils sont ensuite mélangés et répartis en aliquots de 1 ml. Les actifs sont rajoutés à ces aliquots, puis mis à incuber à l'étuve 37°C. Après 24 heures d'incubation, un jury d'experts évalue l'intensité de l'odeur comparativement à un échantillon témoin : 1 ml de sueur incubée sans ajout d'actif.

Les résultats sont exprimés en % de variation de l'intensité de l'odeur comparativement à cet échantillon de sueur témoin (moyenne des % de variation à T24h).

| Actifs testés | Quantité testée (mg MA/ml de sueur | % de diminution de l'intensité de l'odeur |
|---|---|---|
| ACH | 0,1 mg MA | **-37%** |
| (A) | 0,1 mg MA | **- 47%** |
| (B) | 0,4 mg MA | -49% |
| (C) | 0,3 mg MA | -4% |
| ACH + (A) | 0,2 mg MA | **- 72%** |
| ACH + (B) | 0,5 mg MA | -46% |

(suite)

| Actifs testés | Quantité testée (mg MA/ml de sueur) | % de diminution de l'intensité de l'odeur |
|---|---|---|
| ACH + (C) | 0,4 mg MA | -38% |
| ACH : Aluminium chlorohydrate (Micro Dry - Reheis)<br>(A) : Pidolate de zinc (Govobio G Zn - Seppic)<br>(B) : Triclosan (CIBA)<br>(C) : Ricinoleate de zinc (Grillo Werke) | | |

On constate que l'addition du pidolate de zinc au sel d'aluminium conduit à une augmentation substantielle de l'effet de diminution de l'intensité de l'odeur contrairement à l'addition du Triclosan et celle du ricinoléate de zinc au même sel d'aluminium

| Actifs testés | Quantité testée (mg MA/ml de sueur) | % de diminution de l'intensité de l'odeur |
|---|---|---|
| ACH | 0,2 mg MA | **- 47%** |
| (A) | 0,2 mg MA | **- 47 %** |
| 0,1 mg ACH + 0,1 mg (A) | 0,2 mg MA | **- 72 %** |

On constate que l'association pidolate de zinc / sel d'aluminium dans une quantité d'actif totale de 0,2 mg conduit à une augmentation substantielle de l'effet de diminution de l'intensité de l'odeur par rapport à chaque actif utilisé seul à la même quantité. On observe donc une synergie de l'efficacité déodorante.

## II/ Test de microbiologie : activité peu bactéricide du pidolate de zinc par rapport au Triclosan

## Protocole :

Le test décrit ici permet la détermination quantitative de l'activité bactéricide d'une composition sur des germes en conditions optimales de croissance à savoir des germes du type Corynebacterium xerosis (Collection de l'Institut Pasteur n° 5216); Staphylococcus Hominis (Collection de l'Institut Pasteur n° 81 57) et Brevibacterium Epidermidis (Collection de l'Institut Pasteur n° 102110 ) cultivés sur gélose Tryptocaséine soja en pente. La veille du test, dans un pilulier, on dépose 32 g de bouillon Tryptocaséine soja et l'on met à incuber à 35°C. Le jour du test, on ajoute 4 g de la composition à tester et on homogénéise au Vortex.

Un témoin de croissance sans produit est préparé dans les mêmes conditions afin de vérifier que les germes sont dans des conditions de croissance favorables pendant toute la durée du test.

Pour la préparation de l'inoculum, cinq jours avant le début du test, on pratique un repiquage des deux souches bactériennes sur milieu approprié. On incube 5 jours à 35°C. Le jour du test, on lave la pente avec environ 9 ml de diluant. La suspension obtenue titre à 108 germes/ml (on effectue un dénombrement). On introduit 4 ml d'inoculum dans le pilulier, ce qui correspond à un taux de 107 bactéries par gramme de préparation. On place le pilulier dans un incubateur-agitateur (35°C - 200 RPM).

Après chaque temps de contact (2, 4, 6 et 24 heures), on homogénéise le contenu du pilulier au Vortex. On effectue des dilutions décimales. On dépose en surface de boîtes de Pétri gélosée (milieu Eugon LT 100). On incube les boîtes de Pétri 6 à 7 jours à l'étuve à 35°C.

On procède au comptage des colonies sur les boîtes contenant plus de 20 et moins de 200 colonies.

| | |
|---|---|
| résultats identiques : | nulle, |
| 1 Log de réduction : | faible, |
| 2 Log de réduction : | moyenne, |
| 3 Log de réduction : | bonne, |
| ≥ 4 Log de réduction : | excellente. |

On a réalisé d'une part une formule 1 avec le pidolate de zinc et une formule 2 avec le Triclosan .; les supports ont été choisis de manière à être compatibles avec l'actif déodorant.

| Ingrédients | Formule 1 (invention) | Formule 2 (art antérieur) |
|---|---|---|
| Triclosan | | 0,1g |
| Pidolate de zinc | 1 g | |
| Polyéthyleneglycol à 8 OE | 16,8 g | 6g |
| Copolymère réticulé acide acrylique/acrylate d'alkyle en $C_{10}$-$C_{30}$ | - | 0,9g |
| Hydroxyde de sodium pur | - | qs (pH=7) |
| Eau désionisée microbiologiquement propre | 82,8 g | 93,0 g |

On mesure l'activité bactéricide de chacune des formulations 1 et 2 vis à vis des souches *Corynebacterium xerosis, Staphylococcus hominis, Brevibacterium epidermidis* et on l'a compare avec une formule sans actif (placebo). Les résultats obtenus sont résumés dans le tableau suivant :

| Compositions | Efficacité à 24 heures par rapport au gel sans actif | | |
|---|---|---|---|
| Souches | *Corynebacterium xerosis* (CIP 5216) | *Staphylococcus hominis* | *Brevibactérium epidermis* |
| **1 (invention)** | Nulle | Faible | Nulle |
| **2 (art antérieur)** | Excellent | / | / |

On observe que dans la formule 1 , le pidolate de zinc utilisé à une concentration 10 fois plus élevée que celle du Triclosan a une activité antibactérienne faible ou nulle sur les différentes souches testées. Il a donc un spectre d'activité bactéricide sensiblement moins large que celui du Triclosan et respecte d'avantage la flore cutanée.

**III/ Comparaison de l'activité déodorante d'un aérosol anhydre à base de pidolate de zinc en association avec un sel d'aluminium par rapport à un aérosol à base de sel d'aluminium - test in-vivo**

L'amélioration de l'efficacité déodorante du chlorhydrate d'aluminium par association avec le pidolate de zinc a été confirmée in-vivo sur une formule aérosol anhydre antitranspirante. La formule 3 selon l'invention (association d'un pidolate de zinc avec un aluminium chlorhydrate dans un rapport pondéral de 1/9,5) est comparée à une formule 4 ne comprenant qu'un un sel d'aluminium (voir tableau ci-dessous).

| Ingrédients | Formule 3 (invention) | Formule 4 (art antérieur) |
|---|---|---|
| Chlorhydrate d'aluminium (Micro Dry- Reheis) | 5,25g | 5,25g |
| Pidolate de zinc | 0;50 g | - |
| Palmitate d'isopropyle | 0,90 g | 0,90g |
| Tri Ethyl Citrate (Citroflex-Morflex) | 1,05g | 1,05g |
| Cyclopentasiloxane (DC245 -DOW CORNING) | 5,56g | 5,56g |
| Cyclopentasiloxane et Dimethiconol (85,3/14,7) (DC1501 Fluid -DOW CORNING) | 1,35 g | 1,35g |
| Stearalkonium Bentonite (Tixogel MP 250 - Sud Chemie Rheologicals United Catalysts Inc.) | 0,39g | 0,39g |
| Isobutane | 85,00g | 85,00g |

**Protocole du test d'efficacité déodorant:**

On effectue les tests sur 30 volontaires ayant une intensité d'odeur moyenne et symétrique > 5 sur une échelle de 1 (intensité imperceptible) à 9 (intensité extrêmement forte).

Pour chaque volontaire, l'une des aisselles est traitée en une seule application par la formulation 3 , l'autre par la formule 3.

La quantité appliquée est 1.2 ± 0.05 g vaporisé à 15 cm de l'aisselle. Le déodorant est appliqué après essuyage de l'aisselle.

On effectue les évaluations par "sniff test" direct de l'intensité de l'odeur de transpiration et du désagrément de celle-ci , 24 heures après 1 application.

On évalue l'efficacité déodorante par les deux critères suivants :

(1) l'intensité de l'odeur de transpiration (échelle de 1: intensité imperceptible à 9: intensité extrêmement forte) ; plus la valeur est faible moins l'odeur est forte

(2) la valeur hédonique (échelle de 1: odeur extrêmement désagréable à 9: extrêmement agréable) ; plus la valeur est élevée plus le désagrément diminue.

On mesure la variation de l'intensité de l'odeur de la formule 3 selon l'invention contenant l'association pidolate de zinc/sel d'aluminium par rapport à celle de la formule 4 ne contenant pas de pidolate de zinc

$$\% \text{ de variation} = (\text{valeur formule 3} - \text{valeur formule 4}) \times 100 \text{ /formule 4.}$$

| Variation de l'intensité de l'odeur | Variation de la valeur hédonique |
|---|---|
| -14 % | +9 % |

24H après application, les données ont permis de mettre en évidence une diminution significative de l'intensité de l'odeur axillaire et une augmentation significative de la valeur hédonique donc diminution du désagrément pour l'aisselle traitée par la formule 3 selon l'invention contenant l'association pidolate de zinc/sel d'aluminium par rapport à la formule 4 ne contenant pas de pidolate de zinc

**Exemples 5 et 6 : Sticks déodorants**

| Ingrédients | Formule 5 (selon l'exemple 1.5 de la demande WO 01/99376) | Formule 6 (invention) |
|---|---|---|
| Cyclopentasiloxane (DC245 -DOW CORNING) | 23 g | 32 g |
| Hexyldecyl stearate (Eutanol G16 S - COGNIS) | 15g | / |
| PPG-14 Butyl ether (Ucon Fluid AP-Amerchol) | 5 g | 10 g |
| Huile de ricin hydrogénée (Cutina HR-Cognis) | 6 g | 4 g |
| Alcool cétéarylique (Lanette O- Cognis) | 8 g | / |
| Cetearyl alcohol/Ceteareth-30 80/20 (Sinnowax AO-Cognis) | 15g | / |
| Talc | 8g | 2g |
| Aluminium Chlorhydrate (Micro Dry - Reheis) | 20 g | 20 g |
| Pidolate de zinc | 0,05 g | 1 g |
| Alcool stéaryliquel | / | 14 g |

(suite)

| Ingrédients | Formule 5 (selon l'exemple 1.5 de la demande WO 01/99376) | Formule 6 (invention) |
|---|---|---|
| PEG-8 distearate (Distéarate de PEG 400-Stéarines Dubois) | / | 2 g |
| C12-15 alkyl benzoate (Finsolv TN- Witco) | / | 15 g |
| | 100 | 100 |

On chauffe la cyclopentasiloxane à 65°C. On ajoute les autres ingrédients (1 par 1) en restant à 65-70°C. On homogénéise l'ensemble (solution transparente) pendant 15 minutes. On ajoute les 2 actifs déodorants et le talc. On refroidit à environ 55°C (quelques degrés Celcius au dessus de l'épaississement du mélange) et on coule dans les sticks. On met à 4°C pendant 30 minutes.

On mesure selon le test décrit ci-dessous le dépôt de résidu blanc des exemples 3 et 4 après application

### Protocole

La mesure est réalisée sur un appareil de type Minolta CR300. Les produits sont appliqués par frottement en aller-retour jusqu'à obtenir environ 1g de produit pour 40 $cm^2$ sur un papier noir type Canson feuille mi-teinte. La mesure est réalisée immédiatement après application. Une moyenne est faite sur deux mesures.

On mesure un delta L : $\Delta L = L^*$ produit- $L^*$ référence

$L^*$ référence du papier noir type Canson feuille mi teinte : $L^*$ référence = 19.45

On estime qu'un produit est blanchissant et non cosmétiquement acceptable pour un $\Delta L$ supérieur à 35.

Les résultats obtenus sont résumés dans le tableau suivant :

| Composition | $\Delta L$ |
|---|---|
| 5 (art antérieur) | 35 |
| 6 (invention) | 6 |

On constate que la composition 5 selon l'art antérieur contenant l'association pidolate de zinc / chlorhydrate d'aluminium dans un rapport 1/400 produit un taux élevé de résidu blanc sur le substrat tandis que la composition 6 selon l'invention produit un résidu blanc très faible comparable aux sticks déodorants actuellement sur le marché tels que les produits commerciaux "Lady Speed Stick - Clean Glide" de Colgate ou " Secret Clear Dry"de (Procter & Gamble).

### Exemple 7 : Roll-on (émulsion)

| Phase | Ingrédients | Formule 7 |
|---|---|---|
| A | Aluminium Chlorhydrate (50%solution) (Chlorhydrol 50% USP) | 40 g |
| | Pidolate de zinc | 4 g |
| B | Steareth-21 (Brij 721-ICI) | 2g |
| | Steareth-2 (Brij 2 - ICI) | 2g |
| | Steareth-5 Stearate | 1g |
| | PPG-15 stearyl ether (Arlamol E - ICI) | 1,5g |
| | Cyclopentasiloxane (DC245 -DOW CORNING) | 3,5g |
| C | Eau | 47g |
| | | 100g |

On chauffe les phases (B) et (C) séparément à 70°C. On mélange (B) et (C) sous agitation Turax 5min puis refroidir à

EP 1 541 122 B1

55°C sous pâle. On ajoute A doucement en agitant. On homogénéise 1 à 3 minutes. On refroidit à 35°C sous agitation. La formule est stable 2 mois à 45°C.
On mesure le taux de résidu blanc selon le même test décrit dans les exemples 3 et 4. On obtient un ΔL égal à 2.

**Exemple 8 : Spray non aérosol (Emulsion obtenue par inversion de phase)**

| Ingrédients | Formule 8 |
|---|---|
| Aluminium Chlorhydrate (50%solution) (Chlorhydrol 50% USP) | 20g |
| Pidolate de zinc | 3g |
| Cetearyl isononanoate (and) Cetearyl alcohol (and) Ceteareth-20 (and) Glycerin (and) Glyceryl stearate (and) Ceteareth-12 (and) Cetyl palmitate (Emulgade CM- Cognis) | 15g |
| Eau | 63 g |
| | 100g |

On solubilise le pidolate dans l'eau et ajouter à l'Emulgade CM sous agitation modérée. On ajoute la solution de sel d'aluminium sous agitation modérée. La formule est stable 2 mois à 45°C. On mesure le taux de résidu blanc selon le même test décrit dans les exemples 5 et 6. On obtient un ΔL égal à 0.

**Exemple 9 : Aérosol**

| Ingrédients | Formule 9 |
|---|---|
| Stearalkonium Bentonite (Tixogel MP 250 - Sud Chemie Rheologicals United Catalysts Inc.) | 0.5g |
| Aluminium Chlorhydrate (Micro Dry- Reheis) | 7g |
| Pidolate de zinc | 1g |
| $C_{12-15}$ alkyl benzoate (Finsolv TN- Witco) | 3g |
| Isobutane | 80g |
| Tri Ethyl Citrate (Citroflex-Morflex) | 1g |
| Palmitate d'isopropyle | 1g |
| Cyclopentasiloxane (DC245 -DOW CORNING) | 6,5g |
| | 100g |

On introduit les solvants et l'argile modifiée hydrophobe puis on agite au Turax jusqu'à homogénéisation. On ajoute ensuite le sel d'aluminium (anti-transpirant) et le pidolate de zinc tout en agitant. Le propulseur est ensuite introduit de manière classique. On mesure le taux de résidu blanc selon le même test décrit dans les exemples 5 et 6. On obtient un ΔL égal à 4 pour la formule 7.

**Revendications**

1. Composition cosmétique déodorante, comprenant au moins :

   a) le pidolate de zinc et
   b) au moins, à titre de sel d'aluminium anti-transpirant, le chlorhydrate d'aluminium sous forme activée ou non activée; le rapport en poids pidolate de zinc /sel d'Aluminium allant de 1/20 à 5/1.

2. Composition selon l'une quelconque des revendications 1, où le sel d'aluminium anti-transpirant est présente dans des quantités allant de 0,5 à 25% en poids par rapport au poids total de la composition.

3. Composition selon l'une quelconque des revendications 1 à 2 où le pidolate de zinc peut être présent dans des quantités allant de 0,05 à 10% en poids et plus préférentiellement de 0,1 à 5% en poids par rapport au poids total de la composition.

4. Composition, selon l'une quelconque des revendications 1 à 3, **caractérisée par le fait qu'**elle se présente sous forme de lotion, de crème ou de gel fluide distribué en spray aérosol, en flacon pompe ou en roll-on ; sous forme de crème ou gel distribué en tube ou en grille ; sous forme de bâtonnet (stick).

5. Composition selon l'une quelconque des revendications précédentes, **caractérisée par le fait qu'**elle comprend au moins une phase aqueuse.

6. Composition selon la revendication 5, **caractérisée par le fait qu'**elle se présente sous forme de lotion aqueuse, sous forme d'émulsion eau-dans-huile, huile-dans-eau ; sous forme d'émulsion multiple.

7. Composition selon la revendication 6, où la phase aqueuse contient de l'eau et un ou plusieurs solvants solubles ou miscibles dans l'eau.

8. Composition selon la revendication 6, où les solvants solubles ou miscibles dans l'eau sont choisis parmi les mono alcools en $C_1$-$C_4$ ; les diols ; les polyols.

9. Composition selon l'une quelconque des revendications 1 à 4, **caractérisée par le fait qu'**elle est anhydre.

10. Composition selon l'une quelconque des revendications 1 à 9, **caractérisée par le fait qu'**elle comprend au moins une phase liquide organique non-miscible dans l'eau.

11. Composition selon la revendication 10, dans laquelle la phase liquide organique comprend une ou plusieurs huiles émollientes siliconée ou hydrocarbonée, volatiles ou non-volatiles.

12. Composition selon la revendication 11, dans laquelle les huiles émollientes sont présentes dans des quantités allant de 1 à 50% en poids et plus préférentiellement de 5 à 40% en poids par rapport au poids total de la composition.

13. Composition selon l'une quelconque des revendications 1 à 9, **caractérisée par le fait qu'**elle comprend en plus un ou plusieurs actifs déodorants additionnels.

14. Composition selon la revendication 13, **caractérisée par le fait qu'**elle comprend en plus un ou plusieurs agents bactériostatiques ou agents bactéricides.

15. Composition selon l'une quelconque des revendications 1 à 14, **caractérisée par le fait qu'**elle comprend en plus un ou plusieurs agents de suspension.

16. Composition selon l'une quelconque des revendications 1 à 15, **caractérisée par le fait qu'**elle comprend en plus au moins une poudre organique.

17. Composition selon l'une quelconque des revendications 1 à 15, **caractérisée par le fait qu'**elle comprend en plus au moins un additif cosmétique choisi parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs.

18. Composition selon l'une quelconque des revendications 10 à 17, **caractérisée par le fait qu'**elle comprend en plus un ou plusieurs agents structurants ou gélifiants de la phase liquide organique non-miscible dans l'eau.

19. Dispositif aérosol constitué par :

(A) un récipient comprenant une composition déodorante telle que définie selon l'une quelconque des revendications 1 à 18 ;

(B) au moins un agent propulseur et un moyen de distribution de la dite composition aérosol.

20. Procédé cosmétique pour traiter les odeurs axillaires humaines, consistant à appliquer sur la surface axillaire une quantité efficace d'une composition telle que définie telle que définie selon l'une quelconque des revendications 1 à 18.

21. Utilisation de l'association du pidolate de zinc et du chlorhydrate d'aluminium sous forme activée ou non activée dans un rapport en poids allant de 1120 à 5/1 comme actif déodorant dans une composition cosmétique.

**Claims**

1. Deodorant cosmetic composition comprising at least:

   a) zinc pidolate and
   b) at least, as antiperspirant aluminium salt, aluminium chlorohydrate in activated or unactivated form; the zinc pidolate/aluminium salt weight ratio ranging from 1/20 to 5/1.

2. Composition according to Claim 1, in which the antiperspirant aluminium salt is present in amounts ranging from 0.5% to 25% by weight relative to the total weight of the composition.

3. Composition according to either of Claims 1 and 2, in which the zinc pidolate may be present in amounts ranging from 0.05% to 10% by weight and more preferably from 0.1% to 5% by weight relative to the total weight of the composition.

4. Composition according to any one of Claims 1 to 3, **characterized in that** it is in the form of a lotion, a cream or a fluid gel distributed as an aerosol spray, in a pump-dispenser bottle or as a roll-on; in the form of a cream or gel distributed in a tube or grille; in the form of a wand (stick).

5. Composition according to any one of the preceding claims, **characterized in that** it comprises at least one aqueous phase.

6. Composition according to Claim 5, **characterized in that** it is in the form of an aqueous lotion, in the form of a water-in-oil or oil-in-water emulsion or in the form of a multiple emulsion.

7. Composition according to Claim 6, in which the aqueous phase contains water and one or more water-soluble or water-miscible solvents.

8. Composition according to Claim 6, in which the water-soluble or water-miscible solvents are chosen from $C_1$-$C_4$ monoalcohols, diols and polyols.

9. Composition according to any one of Claims 1 to 4, **characterized in that** it is anhydrous.

10. Composition according to any one of Claims 1 to 9, **characterized in that** it comprises at least one water-immiscible organic liquid phase.

11. Composition according to Claim 10, in which the organic liquid phase comprises one or more volatile or non-volatile silicone-based or hydrocarbon-based emollient oils.

12. Composition according to Claim 11, in which the emollient oils are present in amounts ranging from 1% to 50% by weight and more preferably from 5% to 40% by weight relative to the total weight of the composition.

13. Composition according to any one of Claims 1 to 9, **characterized in that** it also comprises one or more additional deodorant active agents.

14. Composition according to Claim 13, **characterized in that** it also comprises one or more bacteriostatic agents or bactericidal agents.

15. Composition according to any one of Claims 1 to 14, **characterized in that** it also comprises one or more suspension agents.

16. Composition according to any one of Claims 1 to 15, **characterized in that** it also comprises at least one organic powder.

17. Composition according to any one of Claims 1 to 15, **characterized in that** it also comprises at least one cosmetic additive chosen from waxes, softeners, antioxidants, opacifiers, stabilizers, moisturizers, vitamins, fragrances, bactericides, preserving agents, polymers, thickeners and propellants.

18. Composition according to any one of Claims 10 to 17, **characterized in that** it also comprises one or more agents for structuring or gelling the water-immiscible organic liquid phase.

19. Aerosol device consisting of:

    (A) a container comprising a deodorant composition as defined according to any one of Claims 1 to 18;
    (B) at least one propellant and a means for distributing the said aerosol composition.

20. Cosmetic process for treating human underarm odours, which consists in applying to the underarm area an effective amount of a composition as defined according to any one of Claims 1 to 18.

21. Use of the combination of zinc pidolate and of aluminium chlorohydrate in activated or unactivated form in a weight ratio ranging from 1/20 to 5/1 as a deodorant active agent in a cosmetic composition.

**Patentansprüche**

1. Desodorierende kosmetische Zusammensetzung, die zumindest enthält:

    a) Zinkpidolat und
    b) Aluminiumchlorhydrat in aktivierter oder nicht aktivierter Form als antitranspiratorisches Aluminiumsalz, wobei das Gewichtsverhältnis Zinkpidolat/Aluminiumsalz 1 /20 bis 5/1 1 beträgt.

2. Zusammensetzung nach Anspruch 1, wobei das transpiratorische Aluminiumsalz in Mengenanteilen von 0,5 bis 25 Gew.-%, bezogen auf das Gesamtgewicht der Zusammensetzung, enthalten ist.

3. Zusammensetzung nach einem der Ansprüche 1 bis 2, wobei das Zinkpidolat in Mengenanteilen von 0,05 bis 10 Gew.-% und noch bevorzugter 0,1 bis 5 Gew.-%, bezogen auf das Gesamtgewicht der Zusammensetzung, enthalten sein kann.

4. Zusammensetzung nach einem der Ansprüche 1 bis 3, **dadurch gekennzeichnet, dass** sie als Lotion, Creme oder fluides Gel, die als Aerosol, Pumpspray oder Roll-on verteilt werden, in Form von Creme oder Gel, die aus einer Tube oder über ein Gitter abgegeben werden; in Form eines Stiftes (Stick) vorliegt.

5. Zusammensetzung nach einem der vorhergehenden Ansprüche, **dadurch gekennzeichnet, dass** sie mindestens eine wässrige Phase aufweist.

6. Zusammensetzung nach Anspruch 5, **dadurch gekennzeichnet, dass** als wässrige Lotion, in Form einer Wasser-in-Öl-Emulsion, Öl-in-Wasser-Emulsion oder Mehrfachemulsion vorliegt.

7. Zusammensetzung nach Anspruch 6, wobei die wässrige Phase Wasser und ein oder mehrere wasserlösliche oder mit Wasser mischbare Lösemittel enthalten.

8. Zusammensetzung nach Anspruch 6, wobei die wasserlöslichen oder mit Wasser mischbaren Lösemittel unter den $C_{1-4}$-Monoalkoholen; Diolen; und Polyalkoholen ausgewählt sind.

9. Zusammensetzung nach einem der Ansprüche 1 bis 4, **dadurch gekennzeichnet, dass** sie wasserfrei ist.

**10.** Zusammensetzung nach einem der Ansprüche 1 bis 9, **dadurch gekennzeichnet, dass** sie zumindest eine nicht mit Wasser mischbare, flüssige organische Phase enthält.

**11.** Zusammensetzung nach Anspruch 10, wobei die flüssige organische Phase ein oder mehrere flüchtige oder nicht-flüchtige, weichmachende Silikonöle oder Kohlenwasserstofföle enthält.

**12.** Zusammensetzung nach Anspruch 11, bei der die weichmachenden Öle in Mengenanteilen im Bereich von 1 bis 50 Gew.-% und vorzugsweise 5 bis 40 Gew.-%, bezogen auf das Gesamtgewicht der Zusammensetzung, enthalten sind.

**13.** Zusammensetzung nach einem der Ansprüche 1 bis 9, **dadurch gekennzeichnet, dass** sie ferner einen oder mehrere, ergänzende desodorisierende Wirkstoffe enthält.

**14.** Zusammensetzung nach Anspruch 13, **dadurch gekennzeichnet, dass** sie ferner einen oder mehrere bakteriostatische Wirkstoffe oder bakterizide Wirkstoffe enthält.

**15.** Zusammensetzung nach einem der Ansprüche 1 bis 14, **dadurch gekennzeichnet, dass** sie ferner ein oder mehrere Suspendiermittel enthält.

**16.** Zusammensetzung nach einem der Ansprüche 1 bis 15, **dadurch gekennzeichnet, dass** sie ferner ein organisches Pulver enthält.

**17.** Zusammensetzung nach einem der Ansprüche 1 bis 15, **dadurch gekennzeichnet, dass** sie ferner mindestens einen kosmetischen Wirkstoff enthält, der unter den Wachsen, beruhigenden Stoffen, Antioxidantien, Trübungsmitteln, Stabilisatoren, Hydratisierungsmitteln, Vitaminen, Parfums, Bakteriziden, Konservierungsmitteln, Polymeren, Parfums, Verdickungsmitteln, Treibmitteln ausgewählt ist.

**18.** Zusammensetzung nach einem der Ansprüche 10 bis 17,
**dadurch gekennzeichnet, dass** sie ferner ein oder mehrere Strukturierungsmittel oder Gelbildner für die nicht mit Wasser mischbare, flüssige organische Phase enthält.

**19.** Aerosolvorrichtung, die aufgebaut ist aus:

(A) einem Behälter, der eine desodorisierende Zusammensetzung enthält, wie sie in einem der Ansprüche 1 bis 18 definiert ist;
(B) mindestens einem Treibmittel und mindestens einer Einrichtung zur Verteilung der Aerosolzusammensetzung.

**20.** Kosmetisches Verfahren für die Behandlung menschlicher Achselgerüche, das darin besteht, eine wirksame Menge einer Zusammensetzung, wie sie in einem der Ansprüche 1 bis 18 definiert ist, auf die Achseloberfläche aufzubringen.

**21.** Verwendung der Kombination aus Zinkpidolat und Aluminiumchlorhydrat in aktivierter oder nicht aktivierter Form in einem Gewichtsverhältnis von 1/20 bis 5/1 als desodorisierenden Wirkstoff in einer kosmetischen Zusammenset-zung.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- DE 10137901
- EP 768080 A
- US 6426061 B
- WO 0152804 A
- WO 0199376 A
- US 4822596 A
- US 4904463 A
- WO 9744010 A

### Littérature non-brevet citée dans la description

- **C. FOX.** *Cosmétics and Toiletries,* Novembre 1986, vol. 101, 101-112